# EUROPÄISCHE PATENTSCHRIFT

(11) **EP 0 725 061 B1**
(45) Veröffentlichungstag und Bekanntmachung des Hinweises auf die Patenterteilung: **07.06.2000**
(21) Anmeldenummer: 96100761.4
(22) Anmeldetag: 19.01.1996
(51) Int. Cl.: C07D 209/18, C07D 209/24, A61K 31/40

(54) **Substituierte Indol-Derivate**
Substituted indole derivatives
Dérivés d'indole substitués

(30) Priorität: 01.02.1995 DE 19503159; 11.04.1995 DE 19513716
(43) Veröffentlichungstag der Anmeldung: 07.08.1996
(73) Patentinhaber: BAYER AG, 51368 Leverkusen (DE)
(72) Erfinder: Müller-Gliemann, Matthias, Dr., D-42697 Solingen (DE); Müller, Ulrich, Dr., D-42111 Wuppertal (DE); Beuck, Martin, Dr., Milford, CT 06460 (US); Zaiss, Sigfried, Dr., D-42113 Wuppertal (DE); Gerdes, Christoph, Dr., D-51373 Leverkusen (DE); Domdey-Bette, Anke, Dr., D-42499 Hückeswagen (DE); Grützmann, Rudi, Dr., D-42657 Solingen (DE); Lohmer, Stefan, Dr., I-20133 Milano (IT); Wohlfeil, Stefan, Dr., D-40724 Hilden (DE); Yalkinoglu, Özkan, Dr., D-42115 Wuppertal (DE); Elting, James, Dr., Madison, CT 06443 (US); Denzer, Dirk, Dr., D-42115 Wuppertal (DE)

(56) Entgegenhaltungen:
- EP-A- 0 397 210
- WO-A-94/18968
- DE-A- 4 304 455
- DE-A- 4 401 893

## Beschreibung

Die Erfindung betrifft Indol-Derivate, Verfahren zu ihrer Herstellung und ihre Verwendung in Arzneimitteln, insbesondere zur Behandlung der Arteriosklerose und der Restenose.

Die DE-A-4304455 offenbart antiartheriosklerotisch wirksame Indolderivate, die in 1-Position mit einer Cyclohexylphenylmethyl-Gruppe substituiert sind. Die EP-A-0397210 beschreibt Indolderivate, die in 2-Position mit substituierten Thiol-Resten substituiert sind und in Therapie und Prophylaxe von Hyperlipidämie und Artheriosklerose Einsatz finden. Die DE-A-4401893 betrifft antiartheriosklerotische Harnstoffderivate, die unter anderem Indolsubstituiert sein können. Ein Verfahren zur Behandlung von Artheriosklerose mit Imidazolderivaten wird in der WO-A-9418968 beschrieben.

Die vorliegende Erfindung betrifft substituierte Indol-Derivate der allgemeinen Formel (I) 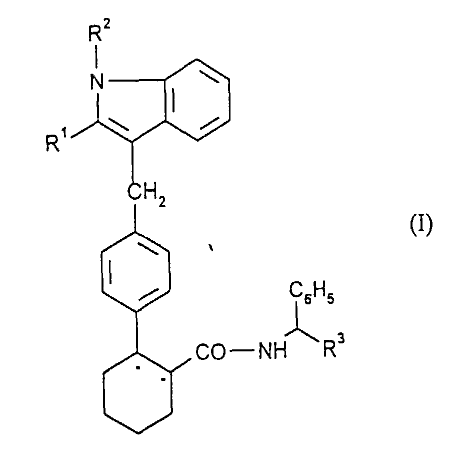 in welcher
- R¹: für Phenyl, Cycloalkyl mit 3 bis 6 Kohlenstoffatomen oder geradkettiges oder für verzweigtes Alkyl mit bis zu 5 Kohlenstoffatomen steht,
- R²: für geradkettiges oder verzweigtes Alkyl mit bis zu 8 Kohlenstoffatomen oder Wasserstoff steht,
- R³: für einen Rest der Formel -CO-NH₂ oder -CH₂-OH steht,
und deren Salze.

Die erfindungsgemäßen substituierten Indol-Derivate können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall-oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher
- R¹: für Phenyl, Cyclopropyl, Cyclopentyl, Cyclohexyl oder geradkettiges oder für verzweigtes Alkyl mit bis zu 4 Kohlenstoffatomen steht,
- R²: für geradkettiges oder verzweigtes Alkyl mit bis zu 6 Kohlenstoffatomen oder Wasserstoff steht,
- R³: für einen Rest der Formel -CO-NH₂ oder -CH₂-OH steht,
und deren Salze.

Besonders bevorzugt sind Verbindungen der allgemeinen Formel (I),
in welcher
- R¹: für Phenyl, Cyclopropyl, Ethyl, iso-Propyl oder n-Butyl steht,
- R²: für geradkettiges oder verzweigtes Alkyl ist mit bis zu 5 Kohlenstoffatomen oder Wasserstoff steht,
- R³: für einen Rest der Formel -CO-NH₂ oder -CH₂-OH steht,
und deren Salze.

Die erfindungsgemäßen Verbindungen der allgemeinen Formel (I) werden hergestellt, indem man Verbindungen der allgemeinen Formel (II) 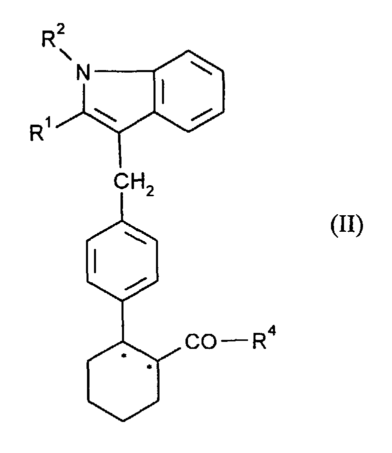 in welcher
- R¹ und R²: die angegebene Bedeutung haben,
und
- R⁴: für geradkettiges oder verzweigtes C₁-C₄-Alkoxy oder Hydroxy stehen
verseift und die Säure, gegebenenfalls unter vorgeschalteter Aktivierung, in inerten Lösemitteln, in Anwesenheit einer Base und/oder Dehydratisierungsmittel mit Phenylglycin-Derivaten der allgemeinen Formel (III) 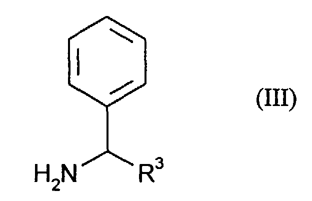 in welcher
- R³: die angegebene Bedeutung hat,
umsetzt.

Das erfindungsgemäße Verfahren kann durch folgendes Formelschema beispielhaft erläutert werden:

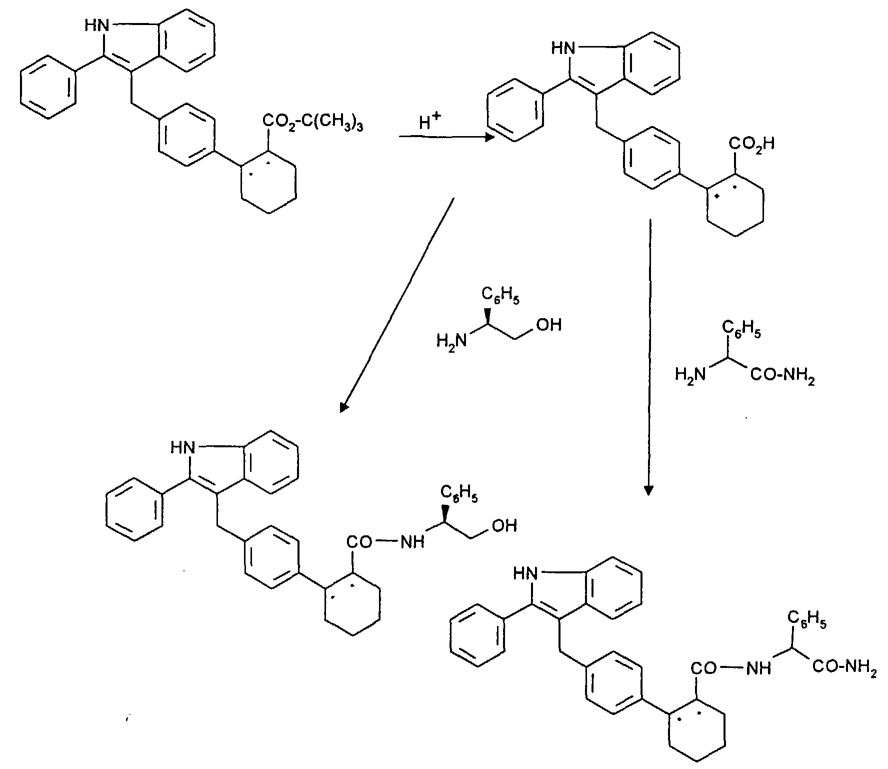

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dichlormethan, Tetrahydrofuran und Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Trimethylamin, Pyridin, Kalium-tert.butylat, DBU oder DABCO.

Im allgemeinen setzt man die Base in einer Menge von 0,05 Mol bis 10 Mol, bevorzugt von 1 Mol bis 2 Mol bezogen auf 1 Mol der Verbindung der Formel (II) ein.

Das erfindungsgemäße Verfahren wird im allgemeinen in einem Temperaturbereich von -50°C bis +100°C, bevorzugt von -30°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Amidierung kann gegebenenfalls über die aktivierte Stufe der Säurehalogenide oder gemischter Anhydride, die aus den entsprechenden Säuren durch Umsetzung mit Thionylchlorid, Phosphortrichlorid, Phosphorpentachlorid, Phosphortribromid oder Oxalylchlorid oder Methansulfonsäurechlorid hergestellt werden können, verlaufen.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die Verbindungen der Formel (II) sind neu und werden hergestellt indem man, Verbindungen der allgemeinen Formel (IV) 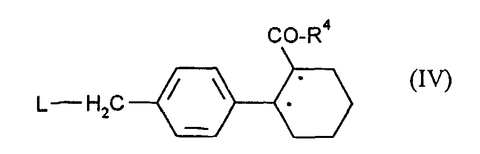 in welcher
- L: für eine typische Abgangsgruppe wie beispielsweise Chlor, Brom, Jod, Tosylat oder Mesylat, vorzugsweise für Brom, steht
und
- R⁴: für geradkettiges oder verzweigtes C₁-C₄-Alkoxy oder Carboxy steht,
zunächst mit Verbindungen der allgemeinen Formel (V) 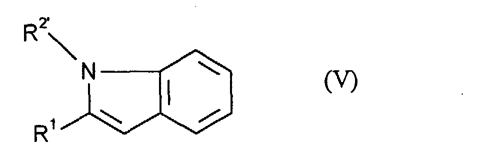 in welcher
- R¹: die oben angegebene Bedeutung hat,
und
- R^{2'}: für Wasserstoff steht,
in inerten Lösemitteln, gegebenenfalls in Anwesenheit einer Base, umsetzt und im Fall R² ≠ H eine Alkylierung nach üblichen Methoden anschließt.

Als Lösemittel, Basen können die oben angegebenen Lösemittel und Basen verwendet werden; bevorzugt sind Dimethylformamid und Kalium-tert.butylat.

Die Alkylierung erfolgt im allgemeinen in einem der oben aufgeführten Lösemittel, vorzugsweise Dimethylformamid mit C₁-C₈-Alkylhalogeniden, vorzugsweise Jodiden, in einem Temperaturbereich von 0°C bis Raumtemperatur und Normaldruck.

Die Verbindungen der Formeln (III), (IV) und (V) sind an sich bekannt.

Die erfindungsgemäßen Verbindungen der allgemeinen Formel (I) zeigen ein nicht vorhersehbares, wertvolles pharmakologisches Wirkspektrum.

Überraschenderweise inhibieren sie die Proliferation von glatten Muskelzellen. Sie können deshalb zur Behandlung der Arteriosklerose und der Restenose eingesetzt werden.

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Schweinen durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% CO₂ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird ³H-Thymidin zugefügt und nach weiteren 4 Stunden der Einbau dieser Substanz in die TCA-präzipitierbare DNA der Zellen bestimmt. Zur Bestimmung der IC₅₀-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

**Tabelle A**

| Beispiel-Nr. | IC₅₀ (nM) |
|---|---|
| 3 | 0,02 |

### Untersuchungen zur Inhibition der c-fos Genexpression glatter Muskelzellen durch die erfindungsgemäßen Verbindungen

Die antiproliferative Wirkung der Verbindungen wurde hinsichtlich der Serum- und Wachstumsfaktor-vermittelten Signaltransduktion und Induktion der c-fos Genexpression in Glattmuskelzell-Reporterlinien untersucht. Als Reporter dient hierbei die Luciferase, dessen Expression über den humanen c-fos Promotor gesteuert wird. Das c-fos Promotor/Luciferasekonstrukt ist stabil in die chromosomale DNA der Ratten-Glattmuskelzelllinie A 10 (ATCC CRL 1476) integriert. Die Reporterzellen werden in 96-Loch-Platten ausgesät und für 1-2 Tage in Serum-haltigem Medium (D-MEM mit 10% FCS, 2 mM L-Glutamin und 15 mM HEPES, pH 7,4) in 5% CO₂ bei 37°C kultiviert. Zur Unterdrückung der c-fos Promotoraktivität auf Basalwerte werden die Zellen durch Serumentzug für 24 Stunden arretiert. Anschließend werden Testverbindungen zugesetzt, und die Zellen mit FCS oder Wachstumsfaktoren zur Induktion der Luciferaseaktivität stimuliert. Nach dieser Behandlungsperiode (4 Stunden) werden die Zellen lysiert und deren Extrakte für die Luciferasebestimmung eingesetzt. Die IC₅₀-Werte werden aus der Wirkstoffkonzentration errechnet, die bei sequentieller Verdünnung des Wirkstoffes die halbmaximale Hemmung der durch den jeweiligen Stimulus hervorgerufene Luciferaseaktivität bewirkt.

### In-vivo-Untersuchungen zur Hemmung der vaskulären Glattmuskelzell-Proliferation im Modell der luftperfundierten Rattencarotis

Die in-vivo-Untersuchungen zur Hemmung der vaskulären Glattmuskelzell-Proliferation im Modell der luftperfundierten Rattencarotis erfolgten nach der leicht modifizierten Methode von Fishman et al. (Lab. Invest. 32, 339-351, 1975); die Operation der Tiere erfolgte unter Nembutal^{R}-Anästhesie. Die rechte Arteria carotis communis wird freigelegt und mit zwei Gefäßklemmen in einem caudal zu cranialen Abstand von ca. 1,5 cm abgeklemmt. Eine Kanüle wird am cranialen Ende dieses Gefäßsegmentes eingeführt, das caudale Ende wird durch den Stich mit einer Nadel perforiert. Nach Spülung mit physiologischer Kochsalzlösung wird ein Luftstrom (25 ml/min für 4 min) durch das Segment perfundiert. Anschließend werden die Klemmen entfernt, die Blutung wird unter leichtem Druck gestillt und das Operationsfeld mit Wundklammern verschlossen. Die Tiere werden acht Tage nach der Operation getötet, und es werden die zuvor luftperfundierten wie auch zur Kontrolle die korrespondierenden contralateralen Carotidensegmente entnommen.
Mit der Applikation der Testsubstanzen (p.o., i.v., i.p., s.c.) wurde zwei Tage vor der Operation begonnen, die Behandlung erfolgte dann über den gesamten Versuchszeitraum (Behandlungsdauer insgesamt: 10 Tage).
Die luftinduzierte Glattmuskelzellproliferation erfolgte mittels Bestimmung des DNA-Gehaltes der Carotidensegmente nach Helms et al. (DNA 43, 39-49, 1985). Dazu werden die Gefäßstücke mit Proteinase K enzymatisch verdaut, die DNA wird isoliert und mit Bisbenzimid fluorometrisch bestimmt (DNA aus Heringssperma als Standard). Der DNA-Gehalt der Gefäße wird schließlich in µg DNA pro mm Carotide angegeben.

Zur Feststellung der antiproliferativen Wirkung der erfindungsgemäßen Verbindungen wird Ratten ein Ballonkatheter in die Halsschlagader eingeführt, dieser aufgeblasen und die Innenseite des Blutgefäßes durch Bewegen des Katheters verletzt [Clowes A.W., et al., Lab. Invest. Vol. 49, No. 3, p. 327, 1983]. Diese Schädigung bewirkt eine neointimale Glattmuskelproliferation, die Stenosen verursacht. Das Ausmaß der Gefäßeinengen bei den Tieren wird nach ca. 2 Wochen durch histologische Aufarbeitung der Blutgefäße bestimmt, indem an Gefäßquerschnitten die Flächen des Proliferationsgewebes vermessen wird.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichtoxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 20mg/kg, vorzugsweise etwa 0,01 bis 5mg/kg Körpergewicht zur Erzielung wirksamer Ergebnisse zu verabreichen, und bei oraler Applikation beträgt die Dosierung etwa 0,01 bis 50mg/kg, vorzugsweise 1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Beispiel I

### trans-2-[4-(2-Phenylindol-3-yl-methyl)-phenyl]cyclohexan-1-carbonsäuretert.butylester

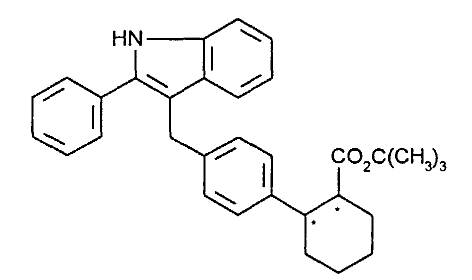

2,8 g (25 mMol) Kalium-tert.butylat werden bei 0°C in 20 ml DMF tropfenweise mit einer Lösung von 5,1 g (25 mMol) 2-Phenylindol versetzt und 30 min gerührt. Anschließend wird eine Lösung von 13,4 g (25 mMol, 60%ig) trans-2-(p-Brommethylphenyl)cyclohexan-1-carbonsäure-tert.butylester in 130 ml DMF innerhalb von 30 min zugetropft und über Nacht auf Raumtemperatur gebracht. Nach Einengen wird in Et₂O/H₂O aufgenommen, vom Niederschlag abgetrennt und dreimal mit Et₂O extrahiert. Nach Trocknen über Na₂SO₄ und Einengen wird das Produkt über Kieselgel 60 (Petrolether / Essigester = 10/1) gereinigt.
- Ausbeute:: 2,21 g (19% d.Th.)
R_{f} = 0,27 (PE / EE = 10/1)

### Beispiel II

### trans-2-[4-(2-Phenylindol-3-yl-methyl)-phenyl]-cyclohexan-1-carbonsäure

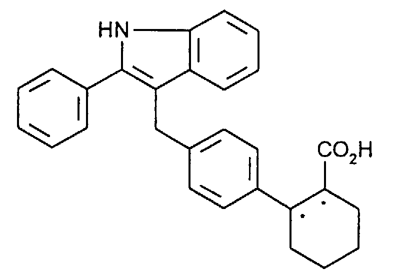

2,2 g (4,7 mMol) der Verbindung aus Beispiel I werden in 15 ml CH₂Cl₂ mit 15 ml Trifluoressigsäure 2 h bei Raumtemperatur gerührt. Nach Einengen wird erneut zweimal mit Et₂O versetzt, eingeengt, in Et₂O wieder aufgenommen, einmal mit 0,5 N NaOH und zweimal mit H₂O (pH 5) extrahiert. Die vereinigten Wasserphasen werden mit 1 N Essigsäure auf pH 4 gestellt und zweimal mit Essigester extrahiert. Die vereinigten Essigesterphasen werden über Na₂SO₄ getrocknet und eingeengt.
- Ausbeute:: 1,8 g (100% d.Th.)
R_{f} = 0,31 (CH₂Cl₂/ / MeOH / NH₃ = 9/1/0,1)

### Beispiel III

### trans-2-[4-(1-Methyl-2-phenylindol-3-yl-methyl)-phenyl]cyclohexan-1-carbonsäuretert.butylester

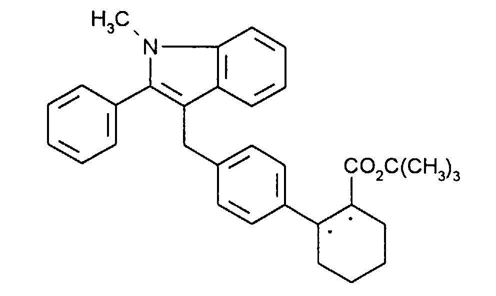

0,4 g (13,2 mmol) NaH (80%) wird in 20 ml DMF suspendiert, auf 0°C gekühlt und tropfenweise mit einer Lösung von 5,6 g (12 mmol) der Ausgangsverbindung aus Beispiel I in 50 ml DMF versetzt. Nach 30 min Rühren werden 2,0 g (14,4 mmol) MeI zugetropft. Nach 1 h Rühren bei 0°C wird auf RT langsam erwärmt und noch 1 h bei dieser Temperatur gerührt. Zur Aufarbeitung wird vorsichtig mit Wasser versetzt und dreimal mit Essigester extrahiert. Die vereinigten organischen Phasen werden über Na₂SO₄ getrocknet, filtriert, eingeengt, und das Produkt wird aus Kieselgel 60 (Petrolether / Essigester = 10/1) chromatographiert.
Ausbeute: 1,2 g (40% d.Th.)
R_{f} = 0.47 (PE/EE = 10/1)

### Herstellungsbeispiele

### Beispiel 1

### trans-2-[4-(2-Phenylindol-3-yl-methyl)phenyl]-cyclohexan-1-carbonyl-(L-phenylglycinolamid)

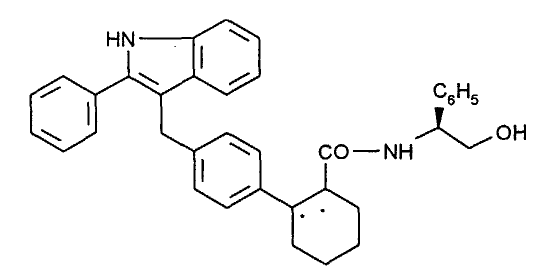

0,14 g (1 mMol) L-Phenylglycinol wird unter Argon in 10 ml CH₂Cl₂ mit 0,41 g (1 mMol) der Verbindung aus Beispiel II und 0,16 g 1-Hydroxy-1H-benzotriazol versetzt, auf -10°C gekühlt, anschließend mit 0,3 ml Triethylamin (2 mMol) und 0,23 g (1,2 mMol) N-(3-Dimethylaminopropyl)-N-ethylcarbodiimid-hydrochlorid versetzt und über Nacht bei Raumtemperatur gerührt. Nach Verdünnen mit CH₂Cl₂ wird mit NH₄Cl, NaHCO₃, H₂O und NaCl extrahiert, über Na₂SO₄ getrocknet, eingeengt und über Kieselgel 60 (CH₂Cl₂ / EtOH = 100/5) gereinigt.
- Ausbeute:: 85,4 mg trans dia B (51,6% d.Th.)
R_{f} = 0,44 (CH₂Cl₂ / MeOH = 95/5)

In Analogie zur Vorschrift des Beispiels 1 wird die in der Tabelle 1 aufgeführten Verbindung hergestellt:

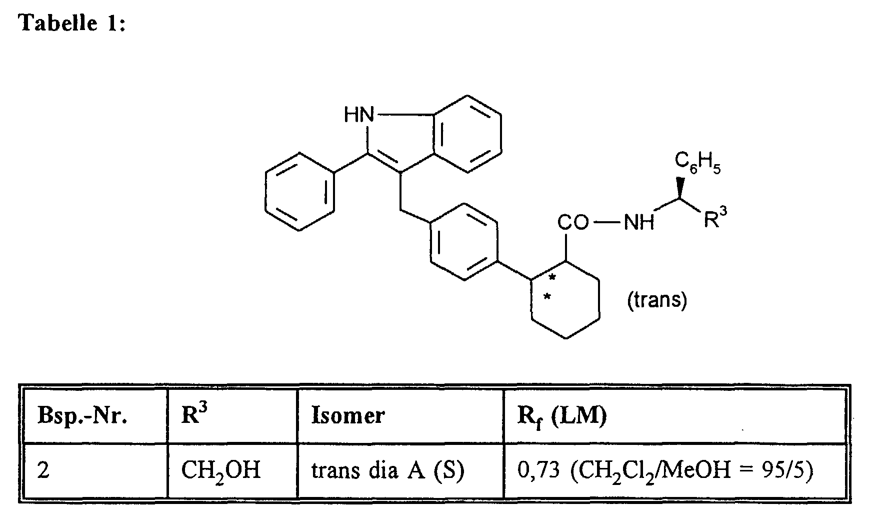

### Beispiel 3

### trans-2-[4-(2-Phenylindol-3-yl-methyl)phenyl]-cyclohexan-1-carbonyl-(phenylglycinamido)amid

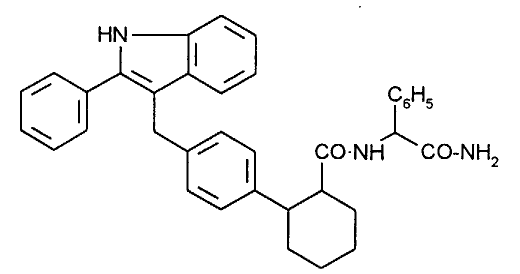

0,16 g (1 mMol) Phenylglycinamid wird in 10 ml CH₂Cl₂ suspendiert, mit 0,41 g (0,1 mMol) der Verbindung aus Beispiel II und 0,16 g (1,1 mMol) 1-Hydroxy-1H-benzotriazol versetzt, auf -10°C abgekühlt und nach Zugabe von 0,3 ml Triethylamin und 0,3 g N-(3-Dimethylaminopropyl)-N-ethylcarbodiimid-hydrochlorid über Nacht bei Raumtemperatur gerührt. Nach Verdünnen mit CH₂Cl₂ wird mit NH₄Cl, NaHCO₃, H₂O und NaCl geschüttelt, die organische Phase über Na₂SO₄ getrocknet, eingeengt und das Produkt über Kieselgel 60 (CH₂Cl₂/EtOH/NH₃ = 100/5/0,1) gereinigt.
- Ausbeute:: 0,29 g trans dia B (51,6% d.Th.)
R_{f} = 0,3 (CH₂Cl₂ / MeOH = 95/5)

In Analogie zur Vorschrift des Beispiels 3 wird die in der Tabelle 2 aufgeführten Verbindung hergestellt:

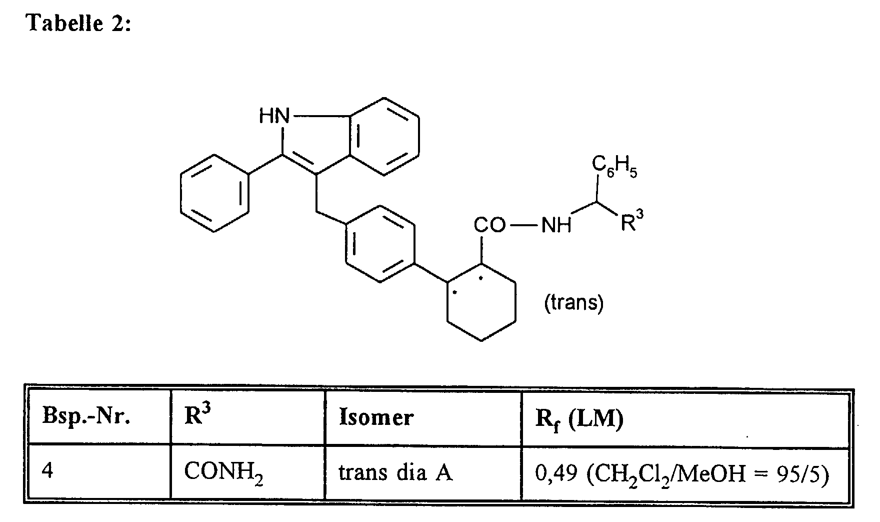

In Analogie zur Herstellung der Beispiele 1 und 2 werden die in der Tabelle 3 aufgeführten Verbindungen hergestellt:

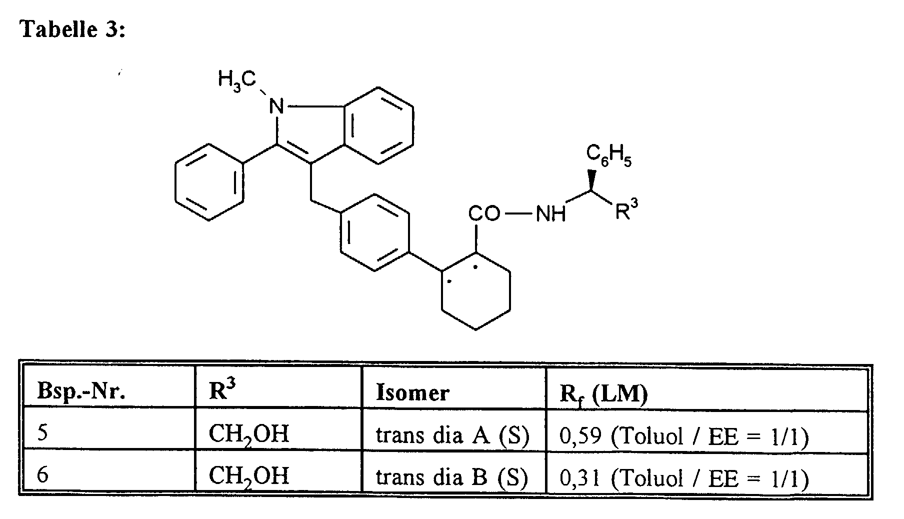

In Analogie zur Herstellung der Beispiele 3 und 4 werden die in der Tabelle 4 aufgeführten Verbindungen hergestellt:

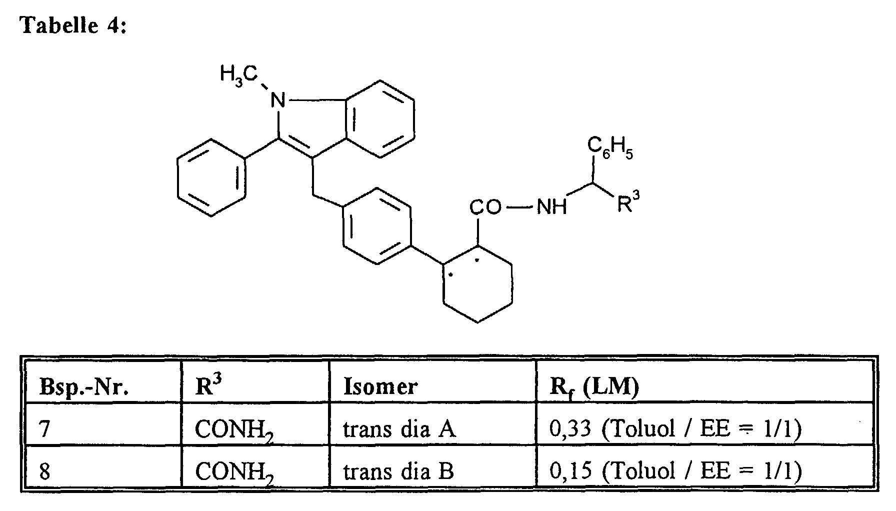

## Patentansprüche

1. Substituierte Indol-Derivate der allgemeinen Formel (I) 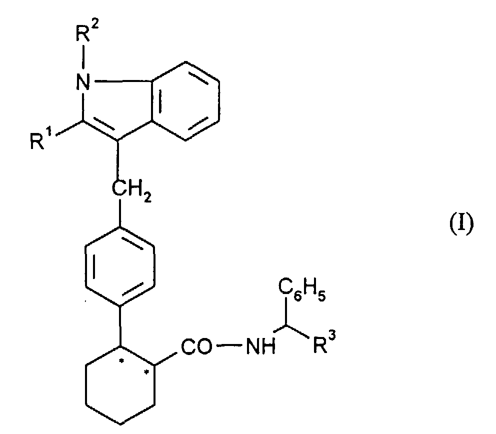 in welcher
R¹ für Phenyl, Cycloalkyl mit 3 bis 6 Kohlenstoffatomen oder geradkettiges oder für verzweigtes Alkyl mit bis zu 5 Kohlenstoffatomen steht,
R² für geradkettiges oder verzweigtes Alkyl mit bis zu 8 Kohlenstoffatomen oder Wasserstoff steht,
R³ für einen Rest der Formel -CO-NH₂ oder -CH₂-OH steht,
und deren Salze.

2. Substituierte Indol-Derivate der Formel (I) nach Anspruch 1
in welcher
R¹ für Phenyl, Cyclopropyl, Cyclopentyl, Cyclohexyl oder geradkettiges oder für verzweigtes Alkyl mit bis zu 4 Kohlenstoffatomen steht,
R² für geradkettiges oder verzweigtes Alkyl mit bis zu 6 Kohlenstoffatomen oder Wasserstoff steht,
R³ für einen Rest der Formel -CO-NH₂ oder -CH₂-OH steht,
und deren Salze.

3. Substituierte Indol-Derivate der Formel (I) nach Anspruch 1
in welcher
R¹ für Phenyl, Cyclopropyl, Ethyl, iso-Propyl oder n-Butyl steht,
R² für geradkettiges oder verzweigtes Alkyl mit bis zu 5 Kohlenstoffatomen oder Wasserstoff steht,
R³ für einen Rest der Formel -CO-NH₂ oder -CH₂-OH steht,
und deren Salze.

4. Substituierte Indol-Derivate nach Anspruch 1 bis 3 zur therapeutischen Anwendung.

5. Verfahren zur Herstellung von substituierten Indol-Derivaten nach Anspruch 1 bis 3, dadurch gekennzeichnet, daß man Verbindungen der allgemeinen Formel (II) 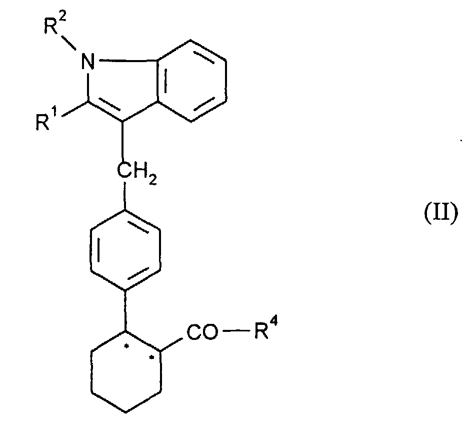 in welcher
R¹ und R² die angegebene Bedeutung haben,
und
R⁴ für geradkettiges oder verzweigtes C₁-C₄-Alkoxy oder Hydroxy stehen
verseift und die Säure, gegebenenfalls unter vorgeschalteter Aktivierung, in inerten Lösemitteln, in Anwesenheit einer Base und/oder Dehydratisierungsmittel mit Phenylglycin-Derivaten der allgemeinen Formel (III) 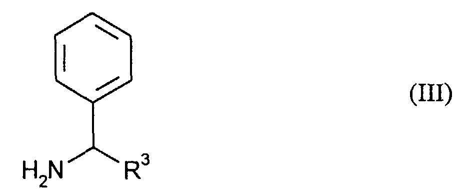 in welcher
R³ die angegebene Bedeutung hat,
umsetzt.

6. Arzneimittel enthaltend mindestens ein substituiertes Indol-Derivat nach Anspruch 1 bis 3.

7. Arzneimittel nach Anspruch 6 zur Behandlung von Arteriosklerose und Restenose.

8. Verfahren zur Herstellung von Arzneimitteln nach Anspruch 6 und 7, dadurch gekennzeichnet, daß man die substituierten Indol-Derivate, gegebenenfalls mit geeigneten Hilfs- und Trägerstoffen in eine geeignete Applikationsform überführt.

9. Verwendung von substituierten Indol-Derivaten nach Anspruch 1 bis 3 zur Herstellung von Arzneimitteln.

10. Verwendung nach Anspruch 9 zur Herstellung von Arzneimitteln zur Behandlung von Arteriosklerose und Restenose.

## Claims

1. Substituted indole derivatives of the general formula (I) 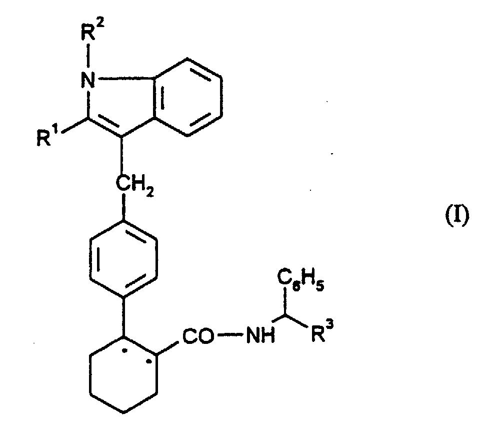 in which
R¹ represents phenyl, cycloalkyl having 3 to 6 carbon atoms or straight-chain or branched alkyl having up to 5 carbon atoms,
R² represents straight-chain or branched alkyl having up to 8 carbon atoms, or hydrogen,
R³ represents a radical of the formula -CO-NH₂ or -CH₂-OH,

2. Substituted indole derivatives according to Claims 1 to 3 for therapeutic use.

3. Process for the preparation of substituted indole derivatives according to Claims 1 to 3, characterized in that compounds of the general formula (II) 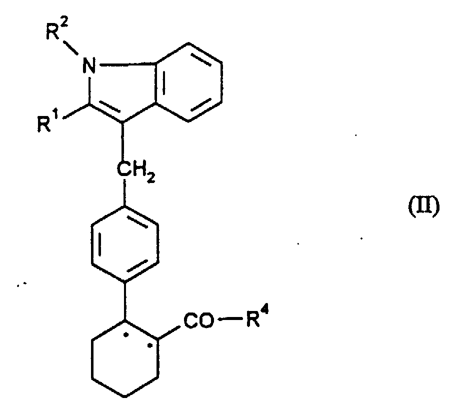 in which
R¹ and R² have the meaning indicated,
and
R⁴ represents straight-chain or branched C₁-C₄-alkoxy or hydroxyl
are hydrolysed and the acid, if appropriate with prior activation, is reacted in inert solvents, in the presence of a base and/or dehydrating agent, with phenylglycine derivatives of the general formula (III) 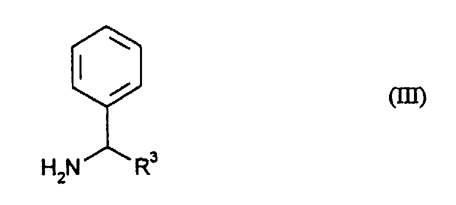 in which
R³ has the meaning indicated.

4. Medicaments containing at least one substituted indole derivative according to Claims 1 to 3.

5. Medicaments according to Claim 6 for the treatment of arteriosclerosis and restenosis.

6. Process for the production of medicaments according to Claims 6 and 7, characterized in that the substituted indole derivatives are converted into a suitable administration form, if appropriate using suitable auxiliaries and excipients.

7. Use of substituted indole derivatives according to Claims 1 to 3 for the production of medicaments.

8. Use according to Claim 9 for the production of medicaments for the treatment of arteriosclerosis and restenosis.

## Revendications

1. Dérivés d'indoles substitués répondant à la formule générale (I) 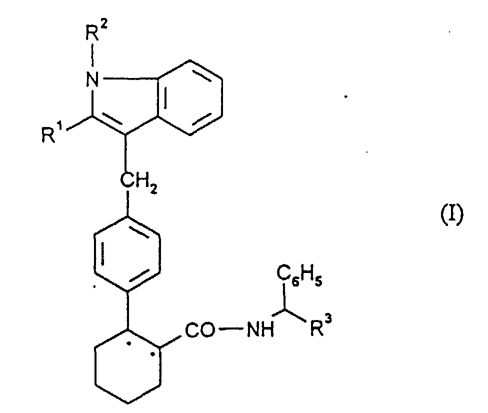 dans laquelle
R¹ représente un groupe phényle, un groupe cycloalkyle contenant de 3 à 6 atomes de carbone ou un groupe alkyle à chaîne droite ou ramifiée contenant jusqu'à 5 atomes de carbone,
R² représente un groupe alkyle à chaîne droite ou ramifiée contenant jusqu'à 8 atomes de carbone ou représente un atome d'hydrogène,
R³ représente un radical répondant à la formule -CO-NH₂ ou -CH₂-OH,
et leurs sels.

2. Dérivés d'indoles substitués répondant à la formule (I) selon la revendication 1,
dans lesquels
R¹ représente un groupe phényle, un groupe cyclopropyle, un groupe cyclopentyle, un groupe cyclohexyle ou un groupe alkyle à chaîne droite ou ramifiée contenant jusqu'à 4 atomes de carbone,
R² représente un groupe alkyle à chaîne droite ou ramifiée contenant jusqu'à 6 atomes de carbone ou représente un atome d'hydrogène,
R³ représente un radical répondant à la formule -CO-NH₂ ou -CH₂-OH,
et leurs sels.

3. Dérivés d'indoles substitués répondant à la formule (I) selon la revendication 1,
dans lesquels
R¹ représente un groupe phényle, un groupe cyclopropyle, un groupe éthyle, un groupe isopropyle ou un groupe n-butyle,
R² représente un groupe alkyle à chaîne droite ou ramifiée contenant jusqu'à 5 atomes de carbone ou représente un atome d'hydrogène,
R³ représente un radical répondant à la formule -CO-NH₂ ou -CH₂-OH,
et leurs sels.

4. Dérivés d'indoles substitués selon les revendications 1 à 3 à des fins d'utilisation thérapeutique.

5. Procédé pour la préparation de dérivés d'indoles substitués selon les revendications 1 à 3, caractérisé en ce qu'on saponifie des composés répondant à la formule générale (II) 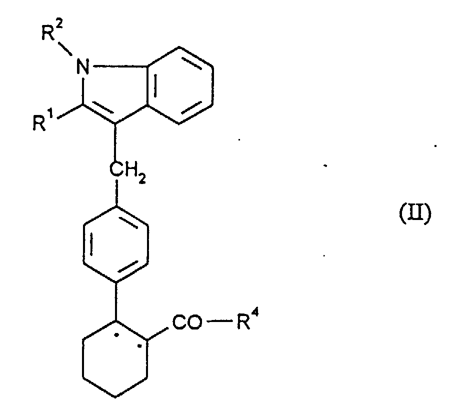 dans laquelle
R¹ et R² ont la signification indiquée,
et
R⁴ représente un groupe alcoxy en C₁-C₄ à chaîne droite ou ramifiée ou un groupe hydroxyle,
et on fait réagir l'acide le cas échéant en procédant à une activation réalisée en amont, dans des solvants inertes, en présence d'une base et/ou d'un agent de déshydratation avec des dérivés de phénylglycine répondant à la formule générale (III) 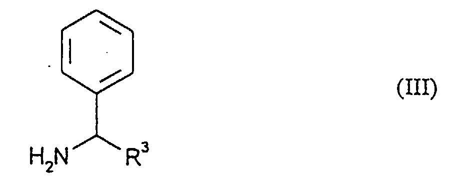 dans laquelle
R³ a la signification indiquée.

6. Médicament contenant au moins un dérivé d'indole substitué selon les revendications 1 à 3.

7. Médicament selon la revendication 6 pour le traitement de l'artériosclérose et de la resténose.

8. Procédé pour la préparation d'un médicament selon les revendications 6 et 7, caractérisé en ce qu'on transforme les dérivés d'indoles substitués, le cas échéant avec des adjuvants et des substances de support appropriés, en une forme d'application appropriée.

9. Utilisation de dérivés d'indoles substitués selon les revendications 1 à 3 pour la préparation de médicaments.

10. Utilisation selon la revendication 9 pour la préparation de médicaments destinés au traitement de l'artériosclérose et de la resténose.
